# EUROPEAN PATENT APPLICATION

(11) **EP 0 661 033 A1**
(43) Date of publication of application: **05.07.1995**
(21) Application number: 94120568.4
(22) Date of filing: 23.12.1994
(51) Int. Cl.: A61F 13/56, A61F 13/58

(54) **Absorbent/mechanical fastener system for absorbent articles**

(30) Priority: 27.12.1993 US 174939
(71) Applicant: KIMBERLY-CLARK CORPORATION, Neenah, Wisconsin 54956-0349 (US)
(72) Inventor: Vukos, John Philip, Neenah, WI 54956 (US)
(74) Representative: DIEHL GLAESER HILTL & PARTNER

(57) **Abstract**

An absorbent article (10, 10', 10'') is disclosed which is designed to be attached to an undergarment. The absorbent article (10, 10', 10'') includes an absorbent having a first end portion (18) and a second end portion (20) separated by a central portion(22), each of the three portions preferably representing about one-third of the absorbent. Attached to the absorbent is a liquid-impermeable baffle (24). Secured to the baffle (24) is an adhesive (31) for holding the absorbent article (10, 10', 10'') securely to the undergarment. The adhesive (31) includes a plurality of spaced apart adhesive members (32) which define a total adhesive surface area. The adhesive members (32) are arranged so that about 35 percent to about 60 percent of the total adhesive surface area is located on the central portion (22).

## Description

This invention relates to an absorbent article which is designed to be attached to an undergarment.

Currently, a wide variety of products for absorption of human body fluids are available in the form of feminine pads, sanitary napkins, panty shields, panty liners and incontinence devices. These products generally have an absorbent positioned between a liquid-permeable bodyside cover and a liquid-impermeable undergarment facing baffle. A pressure-sensitive adhesive which is secured to the baffle is generally used to attach the product to an undergarment's inner crotch portion.

Such adhesively attached absorbent products have met with significant commercial success and have been particularly convenient to use in the days proceeding the woman's menstrual cycle and at the end of the menstrual cycle. Such products also have widespread use during periods of heavy discharge as added protection for a woman using a tampon.

It has been recognized by those skilled in the art that particular placement of the garment attachment adhesive along the undergarment-facing side of the baffle determines, to a large extent, both the effectiveness of the adhesive attachment and the comfort of the article to the wearer. A variety of adhesive placement configurations have been taught in the past. One adhesive configuration includes using one or more bands of adhesive typically placed along the longitudinal axis of the absorbent article. Other configurations include various designs such as an "X", diamonds, chevrons and arcuate strips that are parallel to the article's perimeter. It has been observed that the aforementioned designs generally consist of bands of adhesive which may have unequal length and width dimensions.

It has also been recognized that a sanitary napkin is subjected to side crushing forces by the thighs. These forces tend to permanently distort the napkin along each longitudinal edge. This crushing of the sanitary napkin can, in some cases, not only dislodge the napkin from its secured position, but can also distort the back surface of the napkin. The distortion can cause the centrally disposed adhesive lines to contact each other, preventing the napkin from reattaching to the undergarment.

While there are several different prior art configurations for adhesively securing a sanitary napkin to an undergarment, none of the prior art configurations provide the advantages of the adhesive pattern disclosed herein.

There is, therefore, a need for developing a superior adhesive pattern which would provide secure attachment of a sanitary napkin to the undergarment and provide ease of release without either tearing the napkin or leaving substantial adhesive residue in the undergarment itself.

The invention intends to provide an improved absorbent article which is designed to be attached to an undergarment. This object is solved by the absorbent article of independent claim 1. Further advantageous aspects, features and details of the invention are evident from the dependent claims, the description and the drawings. The claims are intended to understood as a first non-limiting approach of defining the invention in general terms.

The present invention provides an absorbent article which is designed to be attached to an undergarment by a plurality of pressure sensitive adhesive members which define a total adhesive surface area. More specifically, this invention relates to an absorbent having a central portion with about 35 to about 60 percent of the total adhesive surface area being located in this central portion.

The absorbent article includes an absorbent having a first end portion and a second end portion separated by a central portion. Each of the three portions preferably representing about one-third of the absorbent. Attached to the absorbent is a liquid-impermeable baffle. Secured to the baffle is a pressure-sensitive adhesive for holding the absorbent article securely to the undergarment. The adhesive includes a plurality of spaced apart adhesive members which define a total adhesive surface area. The adhesive members are arranged so that about 35 percent to about 60 percent of the total adhesive surface area is located on the central portion.

The general object of this invention is to provide an absorbent article which is designed to be attached to an undergarment by a plurality of adhesive members which define a total adhesive surface area. A more specific aspect of this invention is to provide an absorbent having a central portion with about 35 to about 60 percent of the total adhesive surface area being located in this central portion.

Another aspect of this invention is to provide for an absorbent article that resists displacement by using a combination of adhesive members and nonadhesive frictional members.

Another aspect of this invention is to provide for a sanitary napkin that is easily removed from an undergarment's crotch area.

Other aspects and advantages of the present invention will become more apparent to those skilled in the art in view of the following description and the accompanying drawings.

Fig. 1 is a top perspective view of an absorbent article showing a portion cut away to expose the different layers.

Fig. 2 is a plan view of an absorbent article showing the garment-facing surface of the baffle with a unique adhesive pattern in accordance with this invention.

Fig. 3 is a plan view of an absorbent article showing an alternative embodiment of adhesive pattern.

Fig. 4 is a plan view of an absorbent article showing another alternative adhesive embodiment with adhesive members located adjacent to nonadhesive frictional members.

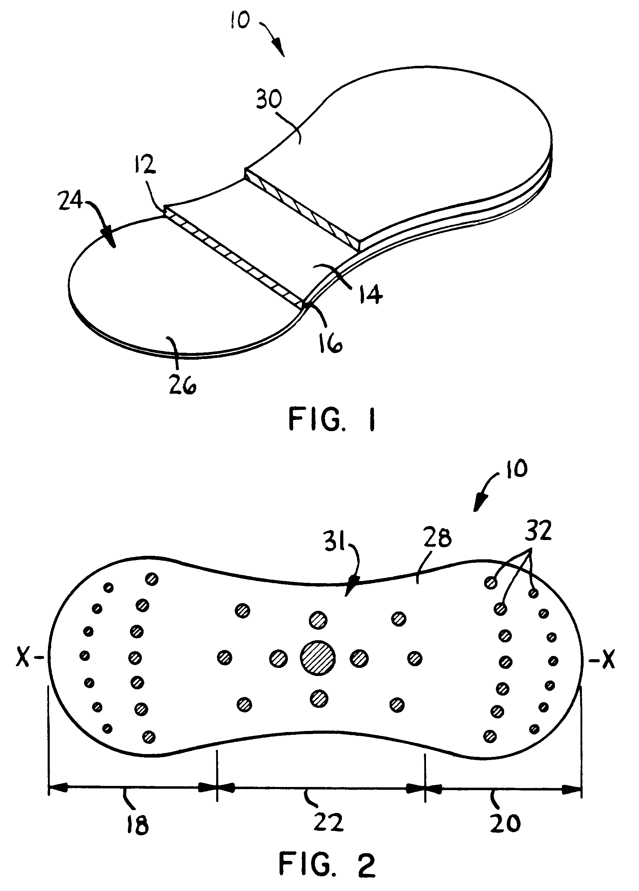

Referring to Figs. 1 and 2, an absorbent article 10 is shown for absorbing human body fluids. For convenience of description only, the absorbent article 10 is illustrated as a panty liner having an hour glass shape but it can be a feminine pad, a sanitary napkin, a panty shield, an incontinence device, or the like. It should be noted that panty liners, feminine pads, and incontinent garments come in various sizes and shapes and vary in thickness. The absorbent article 10 includes an absorbent 12 having a bodyside surface 14 and a garment-facing surface 16. The absorbent 12 has a first end portion 18, a second end portion 20, and a central portion 22 separating the first end portion 18 and the second end portion 20. The absorbent 12 can be made from absorbent materials known in the art such as wood fluff, absorbent polymer filaments such as polypropylene, or a combination of polymer fibers and devilicated wood fibers, polyester or rayon fibers.

A liquid-impermeable baffle 24 is positioned adjacent to the garment-facing surface 16 of the absorbent 12. The liquid-impermeable baffle 24 has an absorbent-facing surface 26 and a garment-facing surface 28, see Fig. 2. The baffle 24 can be made from a polymer material which can permit the passage of air or vapor out of the absorbent article 10 while blocking the passage of body fluids and liquids from the absorbent 12. A good material is a micro-embossed, polymeric film, such as polyethylene or polypropylene. Bi-component films can also be used as well as woven and nonwoven fabrics which have been treated to render them liquid-impermeable. The baffle 24 can be made from a polyethylene film. Most preferably, the baffle 24 is a polyethylene film having a thickness in the range of from about 0.012 mm to about 1.0 mm.

The absorbent article 10 also includes a liquid-permeable cover 30, which is designed to contact the body of the wearer. The liquid-permeable cover 30 is positioned adjacent to at least the bodyside surface 14 of the absorbent 12 and can be secured thereto by adhesives, heat, ultrasonics or a combination thereof. The cover 30 can be constructed of a woven or nonwoven, natural or synthetic material which is easily penetrated by body fluid. Suitable materials include bonded carded webs of polyester, polypropylene, polyethylene, nylon or other heat-bondable fibers. Other polyolefins, such as copolymers of polypropylene and polyethylene, linear low-density polyethylene, finely-perforated film webs and net material, also work well. Particularly preferred are composite materials of a polymer and a nonwoven fabric material. The composite sheets are generally formed by extrusion of the polymer onto a web of spunbonded material to form an integral sheet. This material is preferred, because the outer fabric surface is not irritating to the skin of the wearer. Still another material for the cover 30 is a spunbond web of polypropylene. The web can contain about 1 percent to about 6 percent titanium dioxide pigment to give it a clean, white appearance. The most preferred polypropylene webs have a weight of between about 18 and 40 grams per square meter. An optimum weight is between about 30 and about 40 grams per square meter.

The liquid-permeable cover 30 can also be treated with a surfactant to improve its hydrophilic characteristics and, thereby, aid in the absorption of the liquid. The surfactant can include topical additions or internally applied materials like polysiloxanes.

The absorbent article 10, as illustrated in Figure 1, depicts the baffle 24 and cover 30 configured so that they are coterminous and cooperate to sandwich the absorbent 12 therebetween. Another embodiment of an absorbent article 10 which is equally suitable for incorporating this invention includes an absorbent article 10 wherein the cover 30 over wraps the absorbent 12 and is secured to the baffle 24. Still another embodiment of an absorbent article 10 that is suitable for incorporating this invention is an absorbent article 10 wherein the baffle 24 extends beyond the absorbent 12. In this embodiment, the baffle 24 may partially over wrap the absorbent 12 and cover 30, or the baffle 24 and cover 30 may extend beyond the absorbent 12, cooperating to enclose the absorbent 12 and form a lip.

Secured to the garment-facing surface 28 of the liquid-impermeable baffle 24 is adhesive means 31 for securing the absorbent article to the wearer's undergarment. The adhesive means 31 includes a plurality of similarly configured and spaced apart adhesive members 32. The adhesive members 32 define a total adhesive surface area on the garment-facing surface 28 and are arranged so that about 35 percent to about 60 percent of the total adhesive surface area is located on the central portion 22. Preferably, the total adhesive surface area located on the central portion 22 is from about 35 percent to about 55 percent, and more preferably, from about 40 percent to about 50 percent. The term "total adhesive surface area" as used herein means the mathematical sum of the individual surface areas of the adhesive members 32 present on the garment-facing surface 28. For example, referring to Figure 2, the total adhesive surface area is represented by the shaded area. The adhesive members 32, although shown as having a circular configuration, may be any other geometrical design as long as they are similarly configured. For example, the adhesive members 32 can be square, rectangular, diamond shaped, etc.

Referring to Fig. 2, The adhesive members 32 in the central portion 22 can vary in size. Preferably, the adhesive members 32 in the central portion 22 have from about 1.5 to about 2.0 times greater surface area than the adhesive members 32 in either the first or second end portion 18 and 20, respectively. Thus, a differential attachment force is provided across the garment-facing surface 28 of the baffle 24, with the central portion 22 having the greater adhesive securement. This allows the central portion 22 to remain securely attached to the undergarment while allowing the first end portion 18 and the second end portion 20 to acquire positions on the crotch portion of the undergarment that readily accommodate the distortional forces exerted on the absorbent article 10. This ameliorates the problem, during use, of the absorbent article 10 from adhering to itself instead of the crotch portion of the undergarment after becoming dislodged. This also allows for greater comfort to the wearer.

The adhesive members 32 are provided by applying a pressure-sensitive adhesive composition, known in the garment adhesive art, to the garment-facing surface 28 of the liquid-impermeable baffle 24. The applied adhesive shall have a good tack, good cohesive strength, good resistance to moisture, and good resistance to aging. Illustrative of such adhesive compositions are mixtures of natural or synthetic rubber, various resins or water dispersions of acrylic tacky polymers or copolymers and the like. One suitable garment adhesive is commercially available from National Starch and Chemical Company, located at 10 Finderne Avenue, Bridgewater, New Jersey, 08807.

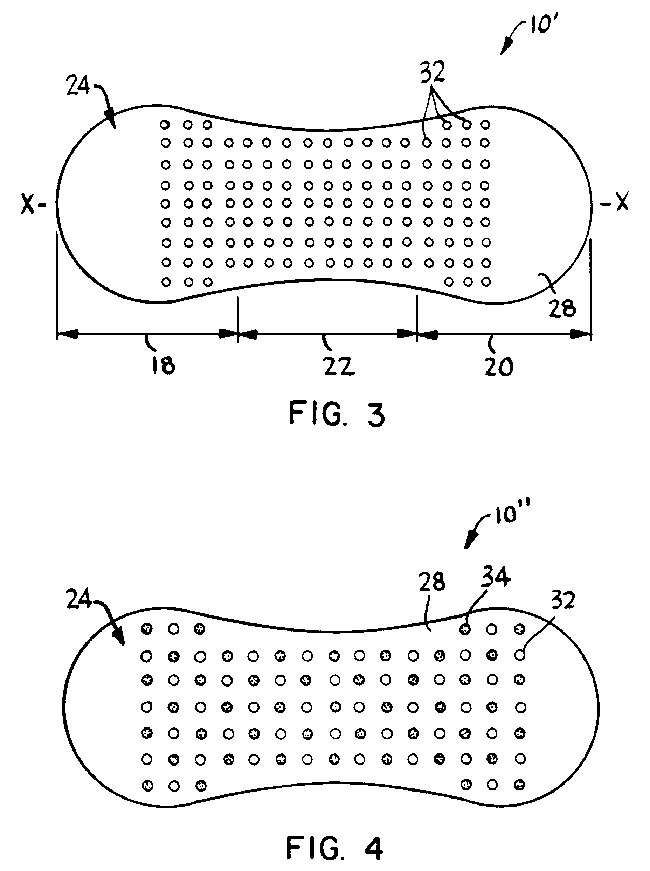

Referring to Fig. 3, an absorbent article 10' is shown having an alternative adhesive pattern wherein a plurality of adhesive members 32 are secured to the garment-facing side 28 of the liquid-impermeable baffle 24. The adhesive members are arranged in a predetermined fashion.

The adhesive members 32 can be longitudinally aligned along the axis X--X of the absorbent article 10'. The adhesive members 32 have an average diameter between about 2 millimeters and about 7 millimeters and can be spaced equidistant apart. Preferably, the adhesive members 32 have an average diameter of between about 2.5 millimeters and about 4.5 millimeters. The adhesive members 32 are arranged so that the central portion 22 of the absorbent article 10' contains from about 35 percent to about 60 percent of the total adhesive surface area. Preferably, the total adhesive surface area located on the central portion 22 is from about 35 percent to about 55 percent, and more preferably, from about 40 percent to about 50 percent.

The density of the adhesive members 32 can be from about 5 to about 30 adhesive members 32 per square centimeter. This density of the adhesive members 32 in the central portion 22 is useful for centrally securing the absorbent article 10' in the crotch portion of the undergarment. This adhesive density in the central portion 22 is also useful in reducing the adverse effects of the crushing forces exerted on the pad during use.

The adhesive members 32 located on the first end portion 18 and the second end portion 20 do not extend to the perimeter of the absorbent article 10'. This provides an area for the wearer to grasp the absorbent article 10' when removing the peel strip (not shown) that generally covers the adhesive members 32. It also provides an area for grasping the absorbent article 10' and removing it from the crotch area of the undergarment for proper disposal.

Referring to Fig. 4, an absorbent article 10'' is shown having an alternative adhesive pattern wherein a plurality of adhesive members 32 are aligned similar to that shown in Fig. 3. The difference is that in Fig. 4 the adhesive members 32 are separated by a plurality of nonadhesive frictional members 34. The nonadhesive frictional members 34 can be of approximately the same size as the adhesive members 32 and can be spaced equidistant apart.

The adhesive members 32 in the central portion 32 occupy an adhesive surface area of about 35 to about 60 percent of the total adhesive surface area. The adhesive members 32 located on the first end portion 18 and the second end portion 20 do not extend to the perimeter of the absorbent article 10'' for the reasons stated above. However, in regards to the nonadhesive frictional members 34, they can extend to the perimeter of the absorbent article 10''. When worn, this arrangement assists in resisting tortious forces exerted on the first and second end portions 18 and 20, respectively. It also reduces the likelihood that the absorbent article 10'' will become adhered to itself if the end portions are dislodged from the crotch of the undergarment.

The nonadhesive frictional members 34 can be formed on the garment-facing side 28 of the liquid-impermeable baffle 24 by using baffle material having a high coefficient of friction, or it can be created by applying a coating to the garment-facing surface 28. Suitable materials include, but are not limited to, various nonwoven fibers and webs, foamed polymers and sponge. As used herein the term "high coefficient of friction" means a coefficient of friction of greater than about 2.0. The coefficient of friction can be determined by ASTM Test No. D-1984, the entire disclosure of which is incorporated herein by reference. The test calls for sled wrapped with Naugahyde to be pulled across the test sample at 5 inches per minute.

An absorbent article is generally equipped with a releasable peel strip (not shown) covering the adhesive members 32. The peel strip protects the adhesive members 32 from prematurely attaching to other items or to one another prior to use. Materials suitable for use as a peel strip can be any material that is capable of being releasably secured to the adhesive members 32. For example, a suitable material is a silicone coated kraft paper, which is commonly used on commercially sold panty liners to protect the garment attachment adhesive prior to use. In use, the peel strip is separated from the absorbent article 10, exposing the adhesive members 32. The wearer then places the absorbent article 10 in the inner crotch portion of the undergarment and pulls the undergarment up to contact the absorbent article 10 with the user.

While the invention has been described in conjunction with several specific embodiments, it is to be understood that many alternatives, modifications and variations will be apparent to those skilled in the art in light of the foregoing description. Accordingly, this invention is intended to embrace all such alternatives, modifications and variations which fall within the spirit and scope of the appended claims.

## Claims

1. An Absorbent article (10, 10', 10'') designed to be attached to an undergarment, said absorbent article (10, 10', 10'') comprising:
a. an absorbent (12) having a first end portion (18) and a second end portion (20) separated by a central portion (22),
b. a liquid-impermeable baffle (24) attached to said absorbent (12), and
c. adhesive means (31) for securing said absorbent article (10, 10', 10'') to said undergarment, said adhesive means (31) including a plurality of spaced apart adhesive members (32) which are positioned on said baffle (24), said adhesive members (32) defining a total adhesive surface area and arranged so that about 35 percent to about 60 percent of said total adhesive surface area is located on said central portion (22).

2. The absorbent article (10, 10', 10'') of claim 1 wherein each of said portions (18, 20, 22) of the absorbent (12) represents approximately one third of said absorbent (12).

3. The absorbent article (10, 10', 10'') of claim 1 or 2, wherein said adhesive means (31) include a plurality of similarly configured adhesive members (32).

4. The absorbent article (10, 10', 10'') of one of the preceding claims, wherein nonadhesive frictional members (34) are formed on said baffle (24).

5. The absorbent article (10, 10', 10'') of claim 4 wherein each of said adhesive members (36) is arranged adjacent to a nonadhesive frictional member (34).

6. The absorbent article (10, 10', 10'') of one of the preceding claims wherein said central portion (22) has about 35 percent to about 55 percent, preferably about 40 percent to about 50 percent, of said total adhesive surface area.

7. The absorbent article (10, 10', 10'') of one of the preceding claims, wherein said plurality of adhesive members (32) is greater than 10.

8. The absorbent article (10, 10', 10'') of one of the preceding claims, wherein each of said adhesive members (32) are circular in configuration.

9. The absorbent article (10, 10', 10'') of one of the preceding claims wherein said adhesive members (32) are equidistance apart.

10. The absorbent article (10, 10', 10'') of one of claims 4 to 9, wherein said adhesive members (32) and said nonadhesive frictional members (34) are approximately the same size.

11. The absorbent article (10, 10', 10'') of one of the preceding claims, wherein said absorbent article further comprises a liquid-permeable cover (30).

12. The absorbent article (10, 10', 10'') of one of the preceding claims, wherein said adhesive members (32) in said central portion (22) have an average diameter between about 2 millimeters and about 7 millimeters, preferably between about 2.5 millimeters and about 4.5 millimeters.

13. The absorbent article (10, 10', 10'') of one of the preceding claims wherein said central portion (22) has a density of between about 5 to about 30 adhesive members (32) per square centimeter.
